Europäisches Patentamt

European Patent Office

Office européen des brevets

(19)

(11) Numéro de publication: **0 532 433 B1**

(12) **FASCICULE DE BREVET EUROPEEN**

(45) Date de publication de fascicule du brevet: **28.06.95** (51) Int. Cl.6: **A61M 1/16**

(21) Numéro de dépôt: **92420300.3**

(22) Date de dépôt: **09.09.92**

(54) **Rein artificiel muni de moyens de détermination de caractéristiques du sang et procédé de détermination correspondant.**

(30) Priorité: **10.09.91 FR 9111352**

(43) Date de publication de la demande:
**17.03.93 Bulletin 93/11**

(45) Mention de la délivrance du brevet:
**28.06.95 Bulletin 95/26**

(84) Etats contractants désignés:
**AT BE CH DE ES FR GB IT LI NL SE**

(56) Documents cités:
**EP-A- 0 097 366**
**EP-A- 0 330 892**
**DE-A- 3 436 748**
**US-A- 4 966 691**

(73) Titulaire: **HOSPAL INDUSTRIE**
**7, Avenue Lionel Terray,**
**BP 126**
**F-69883 Meyzieu Cédex (FR)**

(72) Inventeur: **Bene, Bernard**
**35 rue de la Visine**
**F-69540 Irigny (FR)**

Rank Xerox (UK) Business Services
(3. 10/3.09/3.3.3)

EP 0 532 433 B1

## Description

L'invention concerne un rein artificiel permettant le traitement optimisé de personnes souffrant temporairement ou définitivement d'insuffisance rénale, et plus précisément un rein dont le fonctionnement puisse être réglé en continu en fonction de l'état et des besoins spécifiques de chaque patient, compte tenu des prescriptions du médecin.

On sait que les reins, outre leur fonction d'épuration de certains déchets du métabolisme (urée, créatinine, acide urique, notamment) ont également une fonction d'excrétion hydrique et de régulation de la concentration électrolytique du milieu interne. De sorte que chez les personnes qui ont perdu l'usage de leurs reins, l'organisme se charge en eau, qui se répartit dans les compartiments intra et extracellulaires, en sel et en impuretés, et il est en outre généralement le siège d'un déséquilibre acido-basique.

Le traitement de ces patients se fait au moyen d'un rein artificiel selon l'un ou l'autre, ou encore la combinaison de deux mode de traitement, l'hémodialyse et l'hémofiltration. Le principe de l'hémodialyse consiste à faire circuler, de part et d'autre de la membrane semiperméable d'un échangeur, le sang à épurer et un liquide isotonique au sang, les impuretés migrant du sang vers le liquide de dialyse à un débit d'autant plus élevé que leur concentration dans le sang est importante. Le principe de l'hémofiltration consiste à retirer de l'eau plasmatique du sang par filtration au travers d'une membrane semiperméable, le moteur du transfert étant cette fois la différence des pressions régnant de part et d'autre de la membrane.

La quantité d'eau qu'il faut retirer à un patient chronique lors de chaque séance de dialyse peut être déterminée simplement par une pesée. En revanche, la prescription des différents paramètres de la dialyse (débits du sang et du liquide de dialyse, concentration du liquide de dialyse en électrolytes divers, durée) nécessite une analyse du sang pour en déterminer la concentration en impuretés, celle des électrolytes excédentaires (en général, le sodium, le potassium) et celle des électrolytes déficitaires (en général, le bicarbonate).

De façon classique, l'analyse du sang se fait sur des échantillons prélevés au patient au début et en cours de séance. Cette façon de procéder présente l'inconvénient d'exiger beaucoup de manipulations, donc d'être coûteuse et de faire courir aux personnes chargées des prélèvements et des analyses des risques de contamination accidentelle (hépatite, sida). En conséquence, le nombre de mesures effectuées est nécessairement réduit et ne permet pas toujours d'ajuster la prescription en cours de traitement en vue d'amener le patient, en fin de séance, dans une condition optimale.

Il a donc été proposé des méthodes permettant de déduire certaines caractéristiques du sang de mesures effectuées sur le liquide de dialyse, en particulier sa concentration en substances ionisées, dont le sodium représente la part prépondérante.

En particulier, à partir de l'observation qu'il existe une excellente corrélation entre la conductivité du liquide de dialyse et sa concentration en substances ionisées, il a été développé un procédé de détermination de la natrémie d'un patient consistant à boucler, à intervalles réguliers pendant la séance de dialyse, le circuit de dialyse sur l'échangeur pour y faire recirculer une faible quantité de liquide de dialyse jusqu'à l'équilibre estimé des concentrations de part et d'autre de la membrane. La conductivité du liquide de dialyse est alors mesurée, de laquelle la concentration du sang en substances ionisées, donc en sodium, peut être déduite. Cette méthode présente l'inconvénient de diminuer l'efficacité du traitement pendant les périodes de recirculation.

Par ailleurs, le brevet américain 4 508 622 décrit un dispositif de dialyse permettant de mesurer la différence de conductivité entre le sang et le liquide de dialyse circulant dans un dialyseur et de modifier en cours de séance de traitement la composition du liquide de dialyse de façon que cette différence n'excède pas une valeur déterminée. Ce dispositif comprend deux capteurs similaires déposés en amont et en aval du dialyseur, dont les réponses sont comparées. Il permet donc d'effectuer des mesures aussi fréquentes que souhaité et ne perturbe pas le déroulement du traitement. Cependant, outre qu'il ne fournit pas d'information directe sur la conductivité du sang, il présente plusieurs inconvénients, qui en rendent la mise en oeuvre délicate, tenant essentiellement à la difficulté de maîtriser les dérives non homologues des capteurs, dont les courbes de réponse ne sont par ailleurs jamais identiques, ainsi que de remédier à l'encrassement du capteur aval qui baigne en permanence dans un liquide chargé en substances organiques provenant du plasma.

Une variante de ce dispositif résoud partiellement le problème, un seul capteur étant utilisé, qui est baigné alternativement par du liquide de dialyse frais et du liquide usé prélevés respectivement en amont et en aval de l'échangeur. Cependant, ce dispositif paraît difficilement utilisable sur des reins artificiels équipés d'un dispositif de contrôle volumétrique de l'ultrafiltration où les débits d'entrée et de sortie du circuit de dialyse sont maintenus constants et où une quantité dosée de liquide usé est prélevée en amont de la sortie, correspondant à la quantité d'eau plasmatique que le médecin juge nécessaire de faire perdre au patient. Par ailleurs, la fiabilité de ce dispositif suppose la production d'un liquide de dialyse de composition constante dans le temps, puisque l'échantillon de liquide frais et l'échantillon de liquide usé dont les caractéristiques sont mesurées et comparées ont nécessairement été produits à des instants différents. Il va de soi en effet que, si la concentration en substances ionisées du liquide de dialyse est sujette à des variations dues au mode de production du liquide de dialyse, comme c'est généralement le cas lorsque cette production se fait en ligne, la comparaison des mesures effectuées sur le liquide de dialyse frais et sur le liquide de dialyse usé ne peut fournir que des informations erronées sur la composition du plasma sanguin. Enfin, le prélèvement d'échantillons impose un temps de réponse non négligeable qui ne permet pas de faire de mesures précises sur des solutés chimiquement instables.

Le but de l'invention est d'équiper un rein artificiel de moyens de détermination de caractéristiques du sang qui permettent une mesure fiable et aussi fréquente que souhaité de ces caractéristiques, en vue d'ajuster en permanence le fonctionnement du rein à un objectif thérapeutique fixé par le médecin.

Conformément à l'invention, ce but est atteint au moyen d'un rein artificiel comprenant :

- un échangeur ayant deux compartiments séparés par une membrane semiperméable, un premier compartiment étant relié à un circuit pour circulation extracorporelle de sang, le second compartiment étant relié à un circuit de liquide de dialyse ayant une canalisation d'alimentation en liquide de dialyse frais connectée à une entrée du second compartiment, et une canalisation d'évacuation de liquide usé connectée à une sortie du second compartiment,
- des moyens de mesure pour mesurer au moins une caractéristique du liquide de dialyse frais et du liquide usé,
- des moyens de circulation de liquide de dialyse pour faire balayer en continu les moyens de mesure alternativement avec du liquide de dialyse frais et avec du liquide de dialyse usé, et
- des moyens de calcul pour calculer au moins une caractéristique du sang à partir d'une caractéristique correspondante mesurée dans le liquide de dialyse frais et le liquide usé.

Avantageusement les moyens de circulation de liquide de dialyse comprennent :

- une première dérivation à la canalisation d'alimentation et une seconde dérivation à la canalisation d'évacuation, ces dérivations ayant une portion commune dans laquelle sont disposés les moyens de mesure, et
- des moyens d'occlusion pour permettre la circulation de liquide exclusivement soit dans l'une, soit dans l'autre dérivation.

Grâce à l'agencement des moyens de circulation de liquide de dialyse, il est possible de faire autant de mesures que jugé souhaitable sur le bain de dialyse, sans que l'efficacité du traitement ne soit diminuée ni sans que la durée du traitement ne doive être allongée. Par ailleurs, cet agencement permet de faire balayer les moyens de mesure en permanence par le liquide de dialyse frais, hors les brèves périodes où les caractéristiques du liquide usé sont mesurées, de sorte que l'encrassement des moyens de mesure est évité. En outre, comme les mêmes moyens de mesure sont utilisés pour le liquide de dialyse frais et le liquide usé, d'une part, les mesures sont fiables, et, d'autre part, les mesures peuvent être effectuées sur le même échantillon de liquide de dialyse, avant et après son passage dans l'échangeur, moyennant une temporisation appropriée de l'actionnement des moyens d'occlusion. Enfin, si cet agencement ne permet pas d'éviter toute recirculation de liquide usé, il permet au moins d'en réduire le volume à des valeurs négligeables par rapport au volume total de liquide circulant dans le circuit de dialyse au cours d'une séance de traitement, puisque seul recircule, après chaque mesure sur le liquide usé, le liquide contenu dans la portion de canalisation commune aux dérivations, qu'il est possible de prévoir de très faible contenance.

Selon une caractéristique de l'invention, les moyens de calcul sont également prévus, pour calculer, à partir des informations délivrées par les moyens de mesure, la clairance réelle du rein artificiel pour un type d'impureté donné, telle que l'urée ou la créatinine.

L'invention a également pour objet un procédé de détermination de la concentration d'une substance dans le sang sur un rein artificiel comprenant :

- un échangeur ayant un premier et un second compartiments séparés par une membrane semiperméable et reliés respectivement à un circuit pour circulation extracorporelle de sang et à un circuit de liquide de dialyse et
- des moyens de mesure de la concentration d'au moins une substance dans le liquide de dialyse en amont et en aval de l'échangeur,

    caractérisé en qu'il consiste à :

- faire passer successivement dans le second compartiment deux volumes de liquide de dialyse ayant des concentrations différentes en cette substance, le premier compartiment étant parcouru par du sang,

- mesurer, pour chaque volume de liquide de dialyse, la concentration en cette substance dans le liquide de dialyse en amont et en aval de l'échangeur, et
- calculer, à partir des concentrations mesurées dans les deux volumes de liquide de dialyse, la concentration du sang en cette substance, à l'entrée de l'échangeur.

D'autres caractéristiques et avantages de l'invention apparaîtront à la lecture de la description qui suit. On se reportera aux dessins annexés sur lesquels :

La figure 1 est un schéma simplifié d'un rein artificiel selon l'invention dans un premier mode de fonctionnement ; et

la figure 2 est un détail du rein artificiel représenté sur la figure 1, dans un second mode de fonctionnement.

Le rein représenté sur les figures comprend un échangeur 1 ayant deux compartiments 2, 3 séparés par une membrane 4 semiperméable. Un premier compartiment 2 est relié à un circuit pour circulation extracorporelle de sang comprenant une canalisation amont 5 connectée à une entrée du compartiment 2, sur laquelle est disposée une pompe de circulation 6, et une canalisation aval 7 connectée à une sortie du compartiment 2, comprenant un piège à bulles 8. Les canalisations amont et aval 5, 7 sont munies d'une aiguille à leur extrémité libre pour le raccordement du rein artificiel à un patient 9. Un réservoir 10 contenant un liquide de perfusion stérile est relié au piège à bulles 8 par une canalisation 11 sur laquelle est disposée une pompe de circulation 12.

Le second compartiment 3 de l'échangeur 1 est relié à un circuit de liquide de dialyse comprenant une canalisation d'alimentation 13 connectée, à une extrémité, à une entrée du compartiment 3 et, à son autre extrémité, à une source de liquide de dialyse, et une canalisation d'évacuation 14 de liquide usé connectée, à une extrémité, à une sortie de compartiment 3 et raccordée à l'égout par son autre extrémité. Une pompe de circulation 15 de liquide de dialyse est disposée sur la canalisation d'alimentation 13.

La source de liquide de dialyse comprend un réservoir 16 pour la préparation de liquide de dialyse relié à une source d'eau et à un ou plusieurs réservoirs 17, 18 (deux sur la figure, ce nombre n'étant pas limitatif) contenant des solutions concentrées de différentes compositions. Le débit d'écoulement des solutions concentrées dans le réservoir 16 peut être réglé au moyens de pompes de circulation 19, 20.

Le rein artificiel est muni en outre de moyens de prélèvement et de mesure du filtrat sanguin. Ces moyens comprennent une pompe d'extraction 21 montée pour aspirer du liquide usé dans la canalisation d'évacuation 14 du circuit de liquide de dialyse, tandis qu'une deuxième pompe 22 est disposée sur la canalisation d'évacuation 14, en aval de la pompe d'extraction 21, pour maintenir le débit de sortie du liquide usé égal au débit d'entrée du liquide de dialyse imposé par la pompe de circulation 15. La pompe 22 est régulée par une unité de régulation 23 en fonction de la comparaison des débits d'entrée et de sortie du circuit de dialyse mesurés par deux débitmètres 24, 25 disposés respectivement en amont de la pompe de circulation 15 et en aval de la pompe 22. Le débit d'entrée du liquide de dialyse frais et le débit de sortie du liquide usé étant maintenus égaux, la quantité de liquide usé extraite du circuit de dialyse par la pompe 21 est égale à la quantité d'eau plasmatique filtrée dans l'échangeur, c'est-à-dire à la perte de poids provoquée chez le patient, si celui-ci ne reçoit pas par ailleurs une perfusion de liquide de substitution. Cette quantité d'eau est mesurée par des moyens de mesure volumétrique ou pondéral (non représentés) et la pompe d'extraction 21 est régulée par une unité de commande 26, en fonction de la comparaison entre un débit d'ultrafiltration souhaité $\frac{WL}{T}$ et le débit d'ultrafiltration mesuré.

Conformément à l'invention, ce rein artificiel est muni de moyens de mesure d'une ou plusieurs caractéristiques (conductivité, pH, température, par exemple) du liquide de dialyse et du liquide usé, à partir desquels il est possible de calculer les caractéristiques correspondantes du sang. Ces moyens de mesure comprennent un ou plusieurs capteurs 27 disposés sur une portion de canalisation commune à une première dérivation 28 à la canalisation d'alimentation 13 et à une seconde dérivation 29 à la canalisation d'évacuation 14. Des moyens d'occlusion constitués par trois vannes trois voies 30, 31, 32 sont disposés sur le circuit de liquide de dialyse et ses dérivations pour autoriser la circulation de liquide soit dans l'une soit dans l'autre dérivation exclusivement.

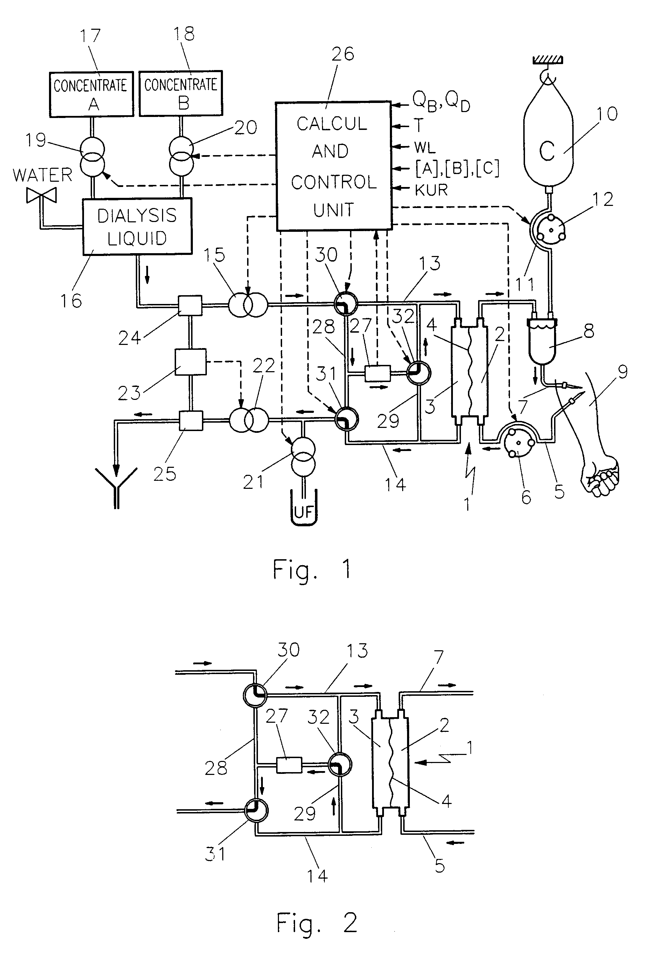

De façon plus détaillée, une première vanne 30 est disposée à la jonction de la canalisation d'alimentation 13 et de l'extrémité amont, par rapport au sens de circulation du liquide de dialyse, de la première dérivation 28. Dans une première position, la vanne 30 contraint le liquide de dialyse frais à circuler dans la première dérivation 28 (figure 1), et, dans une deuxième position, cette vanne coupe la communication entre la canalisation d'alimentation 13 et sa dérivation (figure 2). Une seconde vanne 31 est disposée à la jonction de la canalisation d'évacuation 14 et de l'extrémité aval, par rapport au sens de circulation du liquide usé, de la seconde dérivation 29. Dans une première position, la vanne 31 coupe la communication entre la canalisation d'évacuation 14 et sa dérivation (figure 1) et, dans une deuxième position, cette vanne contraint le liquide usé à circuler de la deuxième dérivation 29 dans la portion de canalisation d'évacuation 14 située en aval de la vanne 31 (figure 2). Une troisième vanne 32 est disposée sur la portion de canalisation commune aux deux dérivations 28, 29, en aval (respectivement en amont) des moyens de mesure 27 par rapport au sens de circulation du liquide de dialyse frais (respectivement du liquide usé). Dans une première position, la vanne 32 interdit la circulation de liquide dans la deuxième dérivation 29 et autorise la circulation de liquide dans la première dérivation 28 (figure 1) et, inversement, dans une deuxième position (figure 2).

En résumé, lorsque les vannes 30, 31, 32 sont dans la première position (figure 1), le liquide de dialyse frais passe dans la première dérivation 28 et balaye les capteurs 27 tandis que le liquide usé circule exclusivement dans la canalisation d'évacuation 14. Et lorsque les vannes sont dans la deuxième position (figure 2), le liquide de dialyse frais circule exclusivement dans la canalisation d'alimentation 13, tandis que le liquide usé passe dans la deuxième dérivation 29 et balaye les capteurs 27.

Conformément à l'invention, les signaux délivrés par les capteurs 27 sont fournis à l'unité de calcul et de commande 26 qui pilote le rein artificiel en fonction de paramètres qu'elle calcule, telles certaines valeurs caractéristiques du sang (concentration en substances ionisées, en bicarbonate, par exemple) ainsi que les performances du rein artificiel (dialysance, clairance pour telle ou telle substance) et en fonction de données qui lui sont fournies par un opérateur préalablement à la séance de traitement, telles que la durée T de la séance, les débits de sang $Q_B$ et de liquide de dialyse $Q_D$, la perte de poids WL souhaitée, la concentration [A], [B], [C] souhaitée dans le sang en électrolytes A, B, C, la clairance en urée $K_{ur}$, souhaitée, notamment.

Le fonctionnement du rein artificiel qui vient d'être décrit est le suivant.

Avant le début d'une séance de traitement, un opérateur fournit à l'unité de calcul et de commande 26 les informations nécessaires au pilotage du rein artificiel, soit la composition du liquide de dialyse en électrolytes A et B, le débit $Q_B$ de la pompe 6 de circulation de sang, le débit $Q_D$ de la pompe 15 de circulation de liquide de dialyse, la perte de poids WL souhaitée et la durée a priori de la séance de traitement T. Ces informations correspondent aux prescriptions du médecin, lesquelles sont établies en fonction de l'état du patient déterminé par une pesée et par une analyse de sang initiales.

En premier lieu, une quantité de liquide de dialyse suffisante pour commencer le traitement est préparée par mélange et chauffage, dans le réservoir 16, d'eau et de solutions concentrées A, B. Le liquide de dialyse peut contenir les principaux électrolytes du sang (sodium, potassium, magnésium, calcium, bicarbonate, chlorure) ou être dépourvu de certains d'entre eux, par exemple de bicarbonate. Dans ce dernier cas, une solution de bicarbonate sera perfusée au patient au cours de la séance de traitement au moyen du dispositif de perfusion 10, 11, 12, pour compenser les pertes diffusives dans l'échangeur 1 et pour, éventuellement, agir sur l'équilibre acido-basique du patient.

Une fois le réservoir 16 rempli de liquide de dialyse prêt à l'emploi et après que les différents circuits du rein artificiel ont été rincés et remplis et que le circuit 5, 7 de circulation extracorporelle de sang a été raccordé au patient 9, toutes les pompes du rein sont mises en marche et/ou réglées aux débits préalablement mis en mémoire dans l'unité 26.

Des mesures vont alors être effectuées sur le liquide de dialyse, à intervalles réguliers, tout au long de la séance de traitement au moyen des capteurs 27. Dans la suite, on prendra l'exemple d'une sonde de conductivité pour expliquer le fonctionnement du rein, le choix de ce capteur particulier ne devant naturellement pas être compris comme limitatif.

Conformément à l'invention, le signal délivré par la sonde de conductivité, dont on a rappelé plus haut qu'il était en étroite corrélation avec la concentration en substances ionisées du liquide qui balaye la sonde, est tout d'abord utilisé pour déterminer la natrémie du patient. Pour ce faire, on programme le passage consécutif dans l'échangeur 1 de deux volumes de liquide de dialyse ayant des conductivités différentes de façon à mesurer, grâce à une bascule des vannes trois voies 30, 31, 32 dans chaque passage de la première à la seconde position, quatre valeurs de conductivité $C_{Di1}$, $C_{Do1}$, $C_{Di2}$, $C_{Do2}$ (respectivement conductivité du liquide de dialyse à l'entrée et à la sortie de l'échangeur, lors du premier passage et lors du second passage), à partir desquelles l'unité 26 peut calculer la concentration $C_{Bi}$ en substances ionisées du sang du patient à l'entrée de l'échangeur, par application de la formule :

$$C_{Bi} = \frac{C_{Do2} \times C_{Di1} - C_{Do1} \times C_{Di2}}{(C_{Di1} - C_{Do1}) - (C_{Di2} - C_{Do2})}$$

cette formule étant déduite de la formule générale de la dialysance D d'un rein artificiel pour un substance considérée qui est définie comme étant égale au rapport entre le transfert de masse pour cette substance $Q_D$ ($C_{Di}$ - $C_{Do}$) et le gradient de concentration de cette substance entre le sang et le bain de dialyse à l'entrée de l'échangeur $C_{Bi}$ - $C_{Di}$.

L'unité 26 compare alors la concentration en substances ionisées du sang du patient (dont le sodium représente la part prépondérante) à la concentration souhaitée préalablement mise en mémoire et commande le cas échéant la pompe 19 ou 20 pour augmenter ou diminuer le débit de la solution concentrée contenant du sodium, c'est-à-dire aussi, toutes choses restant égales par ailleurs, pour augmenter ou diminuer la teneur en sodium du liquide de dialyse.

En outre, conformément à l'invention, l'unité 26 calcule, par extrapolation à partir de la dialysance pour le sodium et selon des règles de correspondance connues, la clairance pour l'urée, qui est une grandeur représentative du rendement épuratif du rein artificiel dépendant à la fois des caractéristiques de l'échangeur (nature et surface de la membrane) et des débits du sang et du liquide de dialyse dans l'échangeur. L'unité 26 compare alors cette clairance pour l'urée calculée à la clairance $K_{ur}$ souhaitée et, le cas échéant, soit modifie la durée de la séance de traitement programmée initialement, soit modifie le débit de la pompe 15 de circulation de liquide de dialyse ou le débit de la pompe 6 de circulation de sang. Dans le premier cas, l'unité 26 modifie le débit de la pompe 21 d'extraction de filtrat sanguin pour tenir compte de l'allongement ou du raccourcissement de la séance de dialyse, la consigne de perte de poids WL restant inchangée par ailleurs.

Lorsque les moyens de mesure 27 comprennent une sonde de pH et de pression partielle de $CO_2$, l'unité 26 calcule la concentration du sang en bicarbonate. Si l'une des solutions concentrées A ou B contient du bicarbonate, l'unité 26 modifie la teneur du liquide de dialyse en bicarbonate en modifiant le débit d'une des pompes 19 ou 20 lorsque la concentration du sang en bicarbonate diffère de la concentration souhaitée. Si le liquide de dialyse ne contient pas de bicarbonate, alors l'apport de bicarbonate au patient se fait par perfusion comme mentionné plus haut, et, lorsque le résultat de la comparaison effectuée par l'unité 26 fait apparaître une différence entre la concentration en bicarbonate mesurée et la concentration souhaitée, l'unité augmente ou réduit le débit de la pompe de perfusion 12.

L'invention n'est pas limitée au mode de réalisation qui vient d'être décrit et elle est susceptible de variantes. En particulier, il va de soi que le fonctionnement du rein artificiel qui vient d'être décrit ne serait pas modifié si les moyens de mesure comprenait au lieu d'une sonde de conductivité et/ou de pH, des détecteurs sélectifs de telle ou telle substance.

## Revendications

1. Rein artificiel comprenant :
   - un échangeur (1) ayant deux compartiments (2, 3) séparés par une membrane semiperméable (4), un premier compartiment (2) étant relié à un circuit (5, 7) pour circulation extracorporelle de sang, le second compartiment (3) étant relié à un circuit de liquide de dialyse (13, 14) ayant une canalisation d'alimentation (13) en liquide de dialyse frais connectée à une entrée du second compartiment (3), et une canalisation d'évacuation (14) de liquide usé connectée à une sortie du second compartiment (3),
   - des moyens de mesure (27) pour mesurer au moins une caractéristique du liquide de dialyse frais et du liquide usé, caractérisé en ce qu'il comprend :
   - des moyens de circulation (28, 29 ; 30, 31, 32) de liquide de dialyse pour faire balayer en continu les moyens de mesure (27) alternativement avec du liquide de dialyse frais et avec du liquide de dialyse usé, et
   - des moyens de calcul (26) pour calculer au moins une caractéristique du sang a partir d'une caractéristique correspondante mesurée dans le liquide de dialyse frais et le liquide usé.

2. Rein artificiel selon le revendication 1, caractérisé en ce que les moyens de circulation de liquide de dialyse comprennent :
   - une première dérivation (28) à la canalisation d'alimentation (13) et une seconde dérivation (29) à la canalisation d'évacuation (14), ces dérivations (28, 29) ayant une portion commune dans laquelle sont disposés les moyens de mesure (27), et
   - des moyens d'occlusion (30, 31, 32) pour permettre la circulation de liquide exclusivement soit dans l'une, soit dans l'autre dérivation.

**3.** Rein artificiel selon la revendication 2, caractérisé en ce que les moyens d'occlusion comprennent trois vannes trois voies (30, 31, 32),

- une première vanne (30) pour établir une communication exclusive entre la canalisation d'alimentation (13) et la première dérivation (28), disposée en amont des moyens de mesure (27) par rapport au sens d'écoulement du liquide de dialyse frais,
- une deuxième vanne (31) pour établir une communication exclusive entre la canalisation d'évacuation (14) et la seconde dérivation (29), disposée en aval des moyens de mesure (27) par rapport au sens d'écoulement du liquide usé, et
- une troisième vanne (32) pour mettre en communication la portion commune aux dérivations (28, 29) soit avec la canalisation d'alimentation (13) soit avec la canalisation d'évacuation (14), disposée en aval (respectivement en amont) des moyens de mesure (27) par rapport au sens d'écoulement du liquide de dialyse frais (respectivement du liquide usé).

**4.** Rein artificiel selon une des revendications 1 à 3, caractérisé en ce que les moyens de mesure (27) comprennent des moyens de mesure de la concentration en au moins une substance (A, B, C).

**5.** Rein artificiel selon la revendication 4, caractérisé en ce qu'il comprend des moyens de commande (26) pour commander, en fonction d'une comparaison entre une concentration ([A], [B]) souhaitée en au moins une substance dans le sang et la concentration calculée en cette substance, des moyens de réglage (19, 20) du débit d'écoulement d'au moins une solution concentrée (A, B) contenant cette substance dans un réservoir (16) de préparation de liquide de dialyse.

**6.** Rein artificiel selon la revendication 4, caractérisé en ce qu'il comprend des moyens (26) pour commander, en fonction d'une comparaison entre une concentration ([C]) souhaitée en au moins une substance (C) dans le sang et la concentration calculée en cette substance, des moyens de réglage (12) du débit d'écoulement d'un liquide de perfusion contenant cette substance (C) dans le circuit (5, 7) pour circulation extracorporelle de sang.

**7.** Rein artificiel selon une des revendications 1 à 6, caractérisé en ce que les moyens de calcul (26) sont prévus également pour calculer, à partir des informations délivrées par les moyens de mesure (27), la clairance réelle du rein artificiel pour un type d'impureté donné, telle que l'urée ou la créatinine.

**8.** Rein artificiel selon les revendications 6 et 7, caractérisé en ce que les moyens de commande (26) sont prévus également pour commander une pompe de circulation (6) disposée sur le circuit (5, 7) pour circulation extracorporelle de sang et/ou une pompe de circulation (15) disposée sur le circuit (13, 14) de liquide de dialyse, en fonction d'une comparaison entre une clairance souhaitée ($K_{UR}$) en un type d'impureté et la clairance calculée.

**9.** Rein artificiel selon une des revendications 7 et 8, caractérisé en ce que les moyens de calcul (26) sont prévus pour calculer, en fonction d'une comparaison entre une clairance souhaitée ($K_{UR}$) en un type d'impureté et la clairance calculée, une durée de séance de traitement, et en ce que les moyens de commande (26) sont prévus en outre pour commander des moyens d'extraction (21) de filtrat sanguin au travers de la membrane (4) en fonction d'une quantité souhaitée de filtrat sanguin (WL) à extraire et de la durée calculée de séance de traitement.

**10.** Rein artificiel selon une des revendications 1 à 9, caractérisé en ce que les moyens de mesure (27) comprennent des moyens de mesure de conductivité.

**11.** Rein artificiel selon une des revendications 1 à 10, caractérisé en ce que les moyens de mesure (27) comprennent des moyens de mesure de pH et de pression partielle de $CO_2$.

**12.** Procédé de détermination de la concentration d'une substance dans le sang d'un patient (9) connecté à un rein artificiel selon une des revendications 1 à 11, caractérisé en ce qu'il consiste à :

- faire passer successivement dans le second compartiment (3) de l'échangeur (1) deux volumes de liquide de dialyse ayant des concentrations différentes en cette substance, le premier compartiment (2) étant parcouru par le sang du patient (9),
- mesurer, pour chacun des deux volumes de liquide de dialyse, la concentration en cette substance dans le liquide de dialyse, en amont et en aval de l'échangeur (1), et

- calculer, à partir des concentrations mesurées dans les deux volumes de liquide de dialyse, la concentration du sang en cette substance, à l'entrée de l'échangeur (1).

**Claims**

1. An artificial kidney comprising :
   - an exchanger (1) having two compartments (2, 3) separated by a semipermeable membrane (4), a first compartment (2) being connected to a circuit (5, 7) for extracorporeal circulation of the blood, the second compartment (3) being connected to a dialysis liquid circuit (13, 14) having a feeder line (13) for a fresh dialysis liquid connected to an inlet of the second compartment (3), and a line (14) for the discharge of used liquid connected to an outlet of the second compartment (3),
   - measurement means (27) for measuring at least one characteristic of the fresh dialysis liquid and of the used liquid, characterised in that it comprises :
   - means (28, 29 ; 30, 31, 32) for circulating dialysis liquid and continuously sweeping the measurement means (27) with fresh dialysis liquid and with used liquid alternately;
   - computing means (26) for calculating a characteristic of the blood from a corresponding characteristic measured in the fresh dialysis liquid and the used liquid.

2. An artificial kidney according to claim 1, characterised in that the means for circulating dialysis liquid comprise :
   - a first branch circuit (28) to the feeder line (13) and a second branch circuit (29) to the discharge line (14), these branch circuits (13, 14) having a common portion wherein the measurement means (27) are disposed, and
   - occluding means (30, 31, 32) for allowing liquid to circulate exclusively either in the one or the other branch circuit.

3. An artificial kidney according to claim 2, characterised in that the occluding means include three three-way valves (30, 31, 32),
   - a first valve (30) for establishing an exclusive communication between the feeder line (13) and the first branch circuit (28), disposed upstream from the measurement means (27) with reference to the direction of flow of the fresh dialysis liquid,
   - a second valve (31) for establishing an exclusive communication between the discharge line (14) and the second branch circuit (29), disposed downstream from the measurement means (27) with reference to the direction of flow of the used liquid, and
   - a third valve (32) for causing the portion common to the branch circuits (28, 29) to communicate either with the feeder line (13) or with the discharge line (14) disposed downstream (or respectively upstream) from the measurement means (27) with reference to the direction of flow of the fresh dialysis liquid (or respectively of the used liquid).

4. An artificial kidney according to one of claims 1 to 3, characterised in that the measurement means (27) comprise means for measuring the concentration of at least one substance (A, B, C).

5. An artificial kidney according to claim 4, characterised in that it comprises control means (26) for actuating, according to a comparison of a desired concentration ([A], [B]) of at least one substance in the blood and the calculated concentration of this substance, means (19, 20) for regulating the flow rate of at least one concentrated solution (A, B) containing this substance into a reservoir (16) for preparing the dialysis liquid.

6. An artificial kidney according to claim 4, characterised in that it comprises means (26) for controlling, according to a comparison of a desired concentration ([C]) of at least one substance (C) in the blood and the calculated concentration of this substance, means (12) for regulating the flow rate of a perfusion liquid containing this substance (C) in the circuit (5, 7) for extracorporeal circulation of the blood.

7. An artificial kidney according to one of claims 1 to 6, characterised in that the computing means (26) are also designed to calculate from the data delivered by the measurement means (27) the real clearance of the artificial kidney for a given impurity such as urea or creatinine.

8. An artificial kidney according to claims 6 and 7, characterised in that the control means (26) are also designed to actuate a circulating pump (6) disposed in the circuit (5, 7) for extracorporeal circulation of the blood and/or a circulating pump (15) disposed in the dialysis liquid circuit (13, 14), according to a comparison of a desired clearance ($K_{UR}$) of a type of impurity and the calculated clearance.

9. An artificial kidney according to one of claims 7 and 8, characterised in that the computing means (26) are designed to calculate a duration of the treatment session according to a comparison of a desired clearance ($K_{UR}$) of a type of impurity and the calculated clearance, and in that the control means (36) are further designed to actuate means (21) for the extraction of blood filtrate through the membrane (4) according to a desired quantity of blood filtrate (WL) to be extracted, and the calculated duration of the treatment session.

10. An artificial kidney according to one of claims 1 to 9, characterised in that the measurement means (27) include means for the measurement of conductivity.

11. An artificial kidney according to one of claims 1 to 10, characterised in that the measurement means (27) include means for the measurement of pH and of the partial pressure of $CO_2$.

12. Method for determining the concentration of a substance in the blood of a patient (9) connected to an artificial kidney according to one of claims 1 to 11, characterised in that it consists in:
   - successively passing two volumes of dialysis liquid having different concentrations of this substance into the second compartment (3) of the exchanger (1), the first compartment (2) being traversed by the blood of the patient (9),
   - measuring for each of the two volumes of the dialysis liquid the concentration of this substance in the dialysis liquid, upstream and downstream from the exchanger (1), and
   - calculating from the concentration measured in the two volumes of dialysis liquid, the concentration of this substance in the blood at the inlet of the exchanger (1).

**Patentansprüche**

1. Künstliche Niere mit:
   - einem Austauscher (1), der zwei Teilräume (2,3) hat, die durch eine halbdurchlässige Membran (4) getrennt sind, wobei ein erster Teilraum (2) mit einem extrakorporellen Blutkreislauf (5,7) verbunden ist, wobei der zweite Teilraum (3) mit einem Dialyseflüssigkeitskreislauf (13,14) verbunden ist, der eine Versorgungsleitung (13) für frische Dialyseflüssigkeit hat, die an einen Einlaß des zweiten Teilraums (3) angeschlossen ist, und eine Abführleitung (14) für gebrauchte Flüssigkeit, die an einen Auslaß des zweiten Teilraums (3) angeschlossen ist,
   - einer Meßeinrichtung (27) zum Messen zumindest einer Eigenschaft der frischen Dialyseflüssigkeit und der benutzten Flüssigkeit,
   dadurch gekennzeichnet, daß sie umfaßt:
   - eine Umwälzeinrichtung (28,29; 30,31,32) für die Dialyseflüssigkeit, um die Meßeinrichtung (27) abwechselnd mit frischer Dialyseflüssigkeit und benutzter Dialyseflüssigkeit durchgehend zu spülen, und
   - eine Berechnungseinheit (26) zum Berechnen zumindest einer Eigenschaft des Bluts ausgehend von einer entsprechenden gemessenen Eigenschaft in der frischen Dialyseflüssigkeit und der benutzten Flüssigkeit.

2. Künstliche Niere nach Anspruch 1, dadurch gekennzeichnet, daß die Umwälzeinrichtung für Dialyseflüssigkeit umfaßt:
   - eine erste Umleitung (28) für die Versorgungsleitung (13) und eine zweite Umleitung (29) für die Abführleitung (14), wobei diese Umleitungen (28,29) einen gemeinsamen Abschnitt haben, in dem die Meßeinrichtung (27) angeordnet ist, und
   - eine Sperreinrichtung (30,31,32) zum ausschließlichen Umwälzen der Flüssigkeit entweder in der einen oder der anderen Umleitung.

3. Künstliche Niere nach Anspruch 2, dadurch gekennzeichnet, daß die Sperreinrichtung drei Dreiwegeventile (30,31,32) umfaßt,

- ein erstes Ventil (30) zum Schaffen einer ausschließlichen Verbindung zwischen der Versorgungsleitung (13) und der ersten Umleitung (28), das stromaufwärts von der Meßeinrichtung (27) in Bezug auf die Strömungsrichtung der frischen Dialyseflüssigkeit angeordnet ist,
- ein zweites Ventil (31) zum Schaffen einer ausschließlichen Verbindung zwischen der Abführleitung (14) und der zweiten Umleitung (29), das stromabwärts von der Meßeinrichtung (27) in Bezug auf die Strömungsrichtung der benutzten Flüssigkeit angeordnet ist, und
- ein drittes Ventil (32) zum Verbinden des gemeinsamen Abschnitts der Umleitungen (28,29) entweder mit der Versorgungsleitung (13) oder mit der Abführleitung (14), das stromabwärts (bzw. stromaufwärts) von der Meßeinrichtung (27) in Bezug auf die Strömungsrichtung der frischen Dialyseflüssigkeit (bzw. der benutzten Flüssigkeit) angeordnet ist.

4. Künstliche Niere nach einem der Ansprüche 1 bis 3, dadurch gekennzeichnet, daß die Meßeinrichtung (27) eine Meßeinrichtung für die Konzentration zumindest einer Substanz (A,B,C) umfaßt.

5. Künstliche Niere nach Anspruch 4, dadurch gekennzeichnet, daß sie eine Steuereinrichtung (26) zum Steuern, als Funktion eines Vergleichs zwischen einer gewünschten Konzentration ([A],[B]) zumindest einer Substanz in dem Blut und der berechneten Konzentration dieser Substanz, eine Regeleinrichtung (19,20) für den Strömungsdurchsatz von zumindest einer konzentrierten Lösung (A,B) umfaßt, die diese Substanz in einem Vorratsbehälter (16) zur Zubereitung der Dialyseflüssigkeit enthält.

6. Künstliche Niere nach Anspruch 4, dadurch gekennzeichnet, daß sie eine Einrichtung (26) zum Steuern, als Funktion eines Vergleichs zwischen einer gewünschten Konzentration ([C]) zumindest einer Substanz (C) in dem Blut und der berechneten Konzentration dieser Substanz, der Regeleinrichtung (12) für die Durchsatzmenge einer Perfusionsflüssigkeit umfaßt, die diese Substanz (C) in dem extrakorporellen Blutkreislauf (5,7) enthält.

7. Künstliche Niere nach einem der Ansprüche 1 bis 6, dadurch gekennzeichnet, daß die Berechnungseinheit (26) außerdem zum Berechnen, ausgehend von Informationen, die durch die Meßeinrichtung (27) geliefert werden, des realen clearance der künstlichen Niere für einen gegebenen Verunreinigungstyp vorgesehen ist, wie beispielsweise Harnstoff oder Kreatinin.

8. Künstliche Niere nach Anspruch 6 und 7, dadurch gekennzeichnet, daß die Steuereinrichtung (26) außerdem zum Steuern einer Umwälzpumpe (6) vorgesehen ist, die in dem extrakorporellen Blutkreislauf (5,7) vorgesehen ist, und/oder einer Umwälzpumpe (15), die in dem Dialyseflüssigkeitskreislauf (13,14) angeordnet ist, als Funktion eines Vergleichs zwischen einer gewünschten clearance ($K_{ur}$) für einen Verunreinigungstyp und der berechneten clearance.

9. Künstliche Niere nach Anspruch 7 und 8, dadurch gekennzeichnet, daß die Berechnungseinheit (26) zum Berechnen, als Funktion eines Vergleichs zwischen einer gewünschten Reinigungsleistung ($K_{ur}$) für einen Verunreinigungstyp und der berechneten Reinigungsleistung, einer Behandlungssitzungsdauer vorgesehen ist, und daß die Steuereinheit (26) außerdem zum Steuern einer Abführeinrichtung (21) für Blutfiltrat durch die Membran (4) als Funktion einer gewünschten abzuführenden Blutfiltratmenge (WL) und der berechneten Dauer für die Behandlungssitzung vorgesehen ist.

10. Künstliche Niere nach einem der Ansprüche 1 bis 9, dadurch gekennzeichnet, daß die Meßeinrichtung (27) eine Leitfähigkeitsmeßeinrichtung umfaßt.

11. Künstliche Niere nach einem der Ansprüche 1 bis 10, dadurch gekennzeichnet, daß die Meßeinrichtung (27) eine Meßeinrichtung für den pH und den partiellen $CO_2$-Druck umfaßt.

12. Verfahren zur Bestimmung der Konzentration einer Substanz im Blut eines Patienten (9), der an eine künstliche Niere gemäß einem der Ansprüche 1 bis 11 angeschlossen ist, dadurch gekennzeichnet, daß es besteht in:
    - aufeinanderfolgendes Strömenlassen von zwei Volumina Dialyseflüssigkeit in dem zweiten Teilraum (3) des Austauschers (1), die unterschiedliche Konzentrationen für diese Substanz haben, wobei der erste Teilraum (2) vom Blut des Patienten (9) durchlaufen ist,
    - Messen der Konzentration dieser Substanz in der Dialyseflüssigkeit stromaufwärts und stromabwärts vom Austauscher (1) für jedes der beiden Dialyseflüssigkeitsvolumina, und

10

- Berechnen der Konzentration des Bluts dieser Substanz am Einlaß des Austauschers (1) ausgehend von gemessenen Konzentrationen in den beiden Dialyseflüssigkeitsvolumina.

Fig. 1

Fig. 2